# FASCICULE DE BREVET EUROPEEN

(11) **EP 1 253 878 B1**
(45) Date de publication et mention de la délivrance du brevet: **08.12.2004**
(21) Numéro de dépôt: 00911003.2
(22) Date de dépôt: 20.03.2000
(51) Int. Cl.: A61F 2/34

(54) **CALOTTE D'ANCRAGE ACETABULAIRE**
HÜFTGELENKVERANKERUNGSPFANNE
ANCHORING ACETABULAR CUP

(43) Date de publication de la demande: 06.11.2002
(73) Titulaire: Ortho - I.D. Sarl, 69006 Lyon (FR); Bonnard, Olivier, 69330 Meyzieu (FR); Bauchu, Philippe, 69002 Lyon (FR); Cypres, Alain, 42300 Roanne (FR); Fiquet, Arnaud, 69300 Caluire (FR); Girardin, Philippe, 42600 Lezigneux (FR); Noyer, Daniel, 38200 Luzinay (FR)
(72) Inventeur: BONNARD, Olivier, F-69330 Meyzieu (FR); BAUCHU, Philippe, F-69002 Lyon (FR); CYPRES, Alain, F-42300 Roanne (FR); FIQUET, Arnaud, F-69300 Caluire (FR); GIRARDIN, Philippe, F-42600 Lezigneux (FR); NOYER, Daniel, F-38200 Luzinay (FR); MOULIN, Jean, F-69006 Lyon (FR)
(74) Mandataire: Brédeville, Odile Marie
(86) Numéro de dépôt international: PCT/FR2000/000688
(87) Numéro de publication internationale: WO 2001/070141

(56) Documents cités:
- EP-A- 0 407 332
- EP-A- 0 532 439
- EP-A- 0 771 552
- EP-A- 0 963 740
- CH-A- 663 893
- FR-A- 2 770 769
- FR-A- 2 783 703
- US-A- 5 658 338

## Description

La présente invention a trait à une calotte d'ancrage dans l'os acétabulaire destinée à servir de surface de glissement pour une coquille articulée sur une prothèse fémorale de hanche.

Un cotyle de fixation acétabulaire selon la technique antérieure comprend une surface sensiblement hémi sphérique d'articulation avec la bille de la prothèse fémorale et une forme extérieure offrant des aspérités ou gorges destinés soit à une fixation par un ciment osseux soit à une fixation par repousse osseuse dans lesdites aspérités.

L'emploi de ciment osseux s'il permet une tenue immédiate des cotyles, présente des inconvénients inhérents aux matériaux: réaction thermique, toxicité du composant de polymérisation et surtout une fragilisation par vieillissement qui créé des particules venant s'interposer entre les surfaces articulaires, et créant de ce fait une usure accélérée conduisant à la réintervention associée parfois à une destruction osseuse.

Les armatures métalliques à ancrage biologique par pénétration osseuse n'ont qu'une faible stabilité initiale et postopératoire surtout lorsque l'os acétabulaire des personnes âgées est de faible résistance. La fixation secondaire définitive n'est obtenue qu'après six à douze semaines pendant lesquelles l'activité du patient doit être modérée.

La présente invention vise à remédier aux inconvénients précités en fournissant un procédé d'ancrage fiable et répétitif sans nécessiter l'utilisation de ciment pour assurer sa fixation quelle que soit la qualité de l'os acétabulaire tout en préservant la qualité du glissement articulaire nécessaire de la prothèse de hanche. Cette invention concerne donc un système constitué d'une calotte d'ancrage dont la surface interne est destinée à servir directement de surface de glissement pour une coquille articulée elle même sur une prothèse fémorale de hanche (page 1 lignes 3 à 5) et c'est donc toute la fonctionnalité, et le but de l'invention qui sont différents de brevets tels que ceux d'IMPACT EP 0 532 439 A, ou TULLOS US 5,658, 338, ou encore NEGRE EP 0 771 552 A.

Cette fixation s'effectuant sans ciment diffère également du document précité TULLOS US 5,658, 338, ainsi que des revendications des brevets OSCOBAL FR 2 197 561 A, HOWMEDICA EP 0 555 004 A

### PRINCIPE DE L'INVENTION :

A cette fin, la calotte d'ancrage acétabulaire présente différents moyens de fixations dans l'os acétabulaire dont la coopération permet de réaliser un ancrage initial immédiat et une tenue à long terme, s'améliorant par l'intégration biologique dans l'os hôte de l'acétabulum et s'opposant parfaitement aux forces alternées d'expulsion générées par les phases d'appui alternatifs lors de la marche.

L'intégration biologique peut être accélérée et augmentée par un traitement de surface tel que corindonage ou par apposition de ciment bio actif sur tout ou partie de la surface en contact avec l'os.

Les dits moyens de fixation sont de nature :
- externes à la cavité acétabulaire grâce à au moins une plaque s'appuyant dans/ou sur le rebord antéro-externe de l'os acétabulaire et dont l'orifice peut recevoir un moyen de stabilisation tel qu'une cheville ou un élément vissé, à titre d'exemple, présentant un système anti recul.
- internes à la cavité acétabulaire grâce à : - au moins un ergot pénétrant l'arrière fond osseux dans la région des cornes postéro inférieures de la dite cavité acétabulaire de manière à en bloquer les mouvements relatifs et permettre l'ostéo - intégration sans interposition fibreuse comme c'est le cas lorsque les cornes restent mobiles, - et des reliefs et macrostructures participant et coopérant à bloquer la calotte, selon les quatre directions de mobilisation possibles : extraction perpendiculaire au plan d'ouverture de la cavité acétabulaire, migration par enfoncement dans l'os, bascule dans le plan d'ouverture et rotation, ainsi que leurs associations. Ces aspérités en relief, font donc saillie par rapport au volume de la calotte d'ancrage, et sont destinées à pénétrer dans l'os lors de la mise en place, et résistent par leur indentation dans l'os à l'arrachement axial et à la bascule qui sont des causes bien connues d'échec par descellement ou de mobilisation précoce de ce type d'implant.

Par exemple :
- l'extraction perpendiculaire est contrôlée par les reliefs annulaires de la zone cylindro - sphérique externe adjacente à l'ouverture inférieure de la calotte,
- la migration par enfoncement est contrôlée par le pole d'appui orienté obliquement par rapport à l'ouverture inférieure de la calotte, mais directement dans l'axe de pression résultant de la charge et de l'action des fessiers,
- la bascule est bloquée par l'effet de rétention créé par la différence d'épaisseur entre la zone inférieure sous l'équateur de la calotte et les reliefs de la zone sus-jacente destinée a l'ajustage serré latéral tangentiel. Le passage en force de cette zone d'ajustage serré se produit par une déformation élastique de l'os acétabulaire, notamment par l'écartement de ses cornes qui après passage de la zone équatoriale se referment et concoure à la fois au maintien en bascule et à la rétention de ladite calotte d'ancrage,
- la rotation est bloquée grâce aux nervures radiales prolongeant le pole d'appui et rejoignant les reliefs annulaires ;

L'orientation des dites nervures est prolongée dans la réalisation des crantages verticaux définissant les dits reliefs annulaires.

L'ensemble de cette architecture créé une direction d'introduction privilégiée, oblique par rapport au plan de l'ouverture inférieure de la cavité acétabulaire et dans l'axe global de la zone d'appui polaire, donc dans l'axe des forces exercées.

### DESCRIPTION:

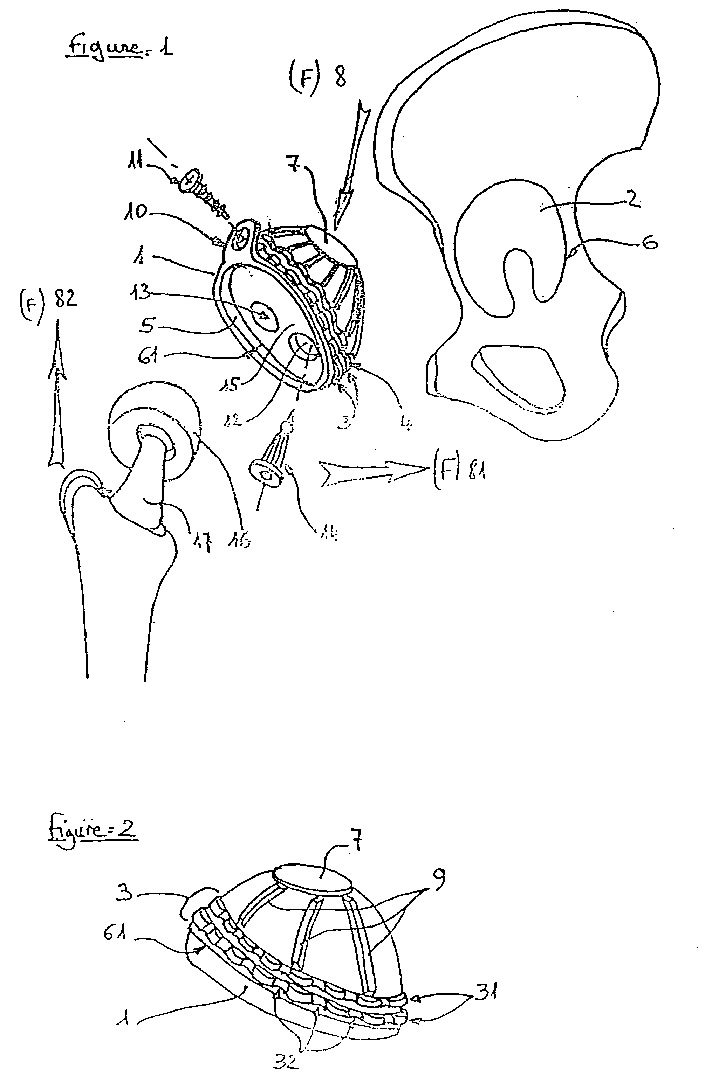

La figure 1 est une vue supéro antérieure montrant l'aspect externe global de la calotte d'ancrage (1) destinée à servir de surface de glissement pour une coquille (16) articulée sur une prothèse fémorale (17) représentant la partie de contact annulaire (3) formée par la zone cylindro sphérique (5), la partie d'appui polaire (7) oblique par rapport à l'équateur (61) de ladite calotte (1), les nervures (9) anti-rotation rayonnant à partir de la dite partie d'appui polaire (7), une plaque (10) disposée radialement à l'équateur (61) munie d'au moins un orifice (100) recevant un moyen de fixation (11), et un orifice (12) traversant ladite calotte (1) muni d'un ergot (14) dirigé vers la partie postéro inférieure pour s'ancrer dans les cornes de la cavité acétabulaire (2).

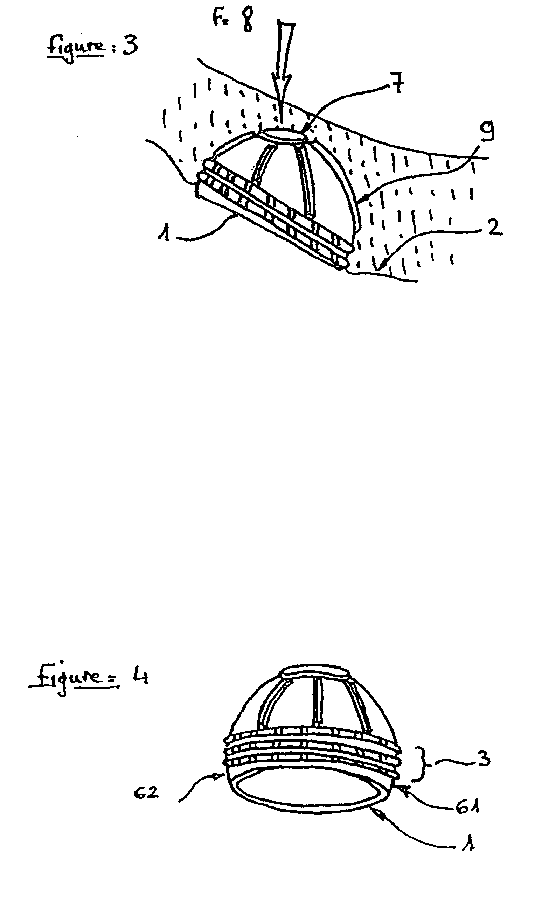

La figure 2 représente une vue de détail d'un mode de réalisation de la partie de contact annulaire (3) avec des bandes de relief (31) sensiblement parallèles à l'équateur (61) de ladite calotte (1), et des gorges (32) sensiblement perpendiculaires à l'équateur (61), pouvant dans un mode de réalisation préférentiel être dans la direction du pole d'appui oblique (7) de manière à prolonger l'effet directif des nervures (9).

La figure 3 représente un mode de réalisation des nervures (9) et leurs positions radiales autour du pole dans la direction de la résultante des forces musculaires et d'appui (8) avec l'effet de relief destiné à faire pénétrer les dites nervures (9) dans l'os acétabulaire (2) lors de l'impaction en force de la dite calotte (1).

De préférence, les nervures (9) sont en relief par rapport à la sphère théorique et dépassent du pole d'appui (7).

La figure 4 montre la différence de diamètre entre la zone d'ajustage serré tangentiel réalisée par la partie de contact annulaire (3) cylindro sphérique immédiatement au-dessus de l'équateur (61), et la zone lisse (62) en dessous de l'équateur dont le retrait permet au rebord osseux de se refermer et d'emprisonner la calotte d'ancrage (1).

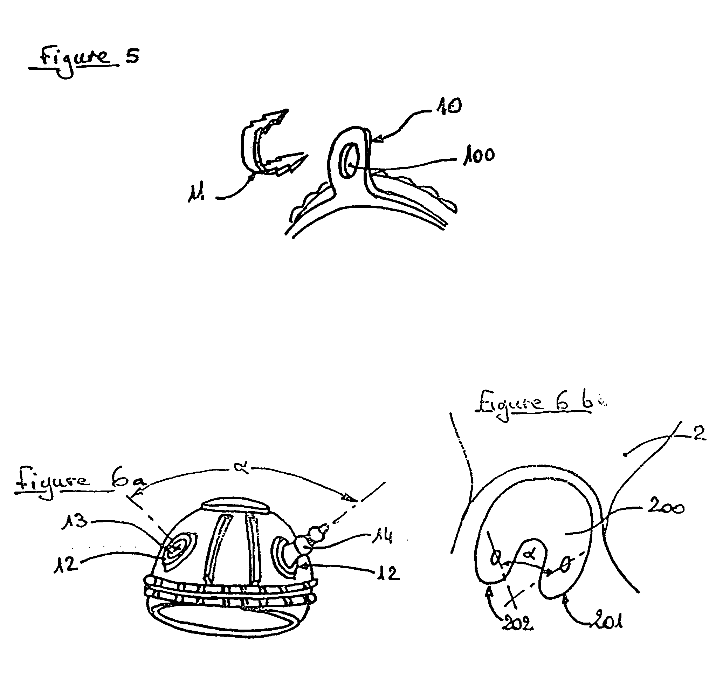

La figure 5 est une vue de la plaque (10) recevant au moins un orifice (100) et à titre d'exemple un mode de réalisation du moyen de fixation (11) destiné à y être introduit.. De préférence ce moyen de fixation peut être conçu pour ne pas reculer une fois introduit dans le dit orifice (100). La calotte d'ancrage (1) dans l'os acétabulaire (2) présente au moins une plaque (10) disposée à la périphérie de la dite calotte (1), de préférence à distance de son plan d'ouverture (6), à la jonction de la zone cylindrique lisse (62) et de la zone de contact annulaire (3), afin d'augmenter la stabilité des pièces articulaires fémorales (16), par le débord osseux de ladite zone lisse (62) en dessous de l'équateur. Cette plaque est de préférence inclinée vers le pôle d'appui (7) de manière à s'adapter au rebord osseux de la cavité acétabulaire (2) sans besoin de modelage in situ.

Les figures 6 et 6bis représentent à titre d'exemple un ergot (14) dans un orifice (12) et un obturateur (13) dans l'autre orifice (12) ainsi que leurs positions respectives par rapport aux cornes postéro inférieures (201 et 202) qu'elles contribuent à bloquer.

La calotte d'ancrage (1) dans l'os acétabulaire (2) présente sur la face convexe d'ancrage osseux une apposition de revêtement bio actif pour accélérer le processus de cicatrisation des tissus osseux périphériques.

La calotte d'ancrage (1) dans l'os acétabulaire (2) peut présenter sur la fàce convexe d'ancrage osseux un traitement de surface de préférence agressif, du type corindonage à titre d'exemple, pour permettre l'attachement de l'os périphérique.

La calotte d'ancrage (1) dans l'os acétabulaire (2) peut comprendre au moins 2 ergots (14) pénétrant dans les cornes (201 et 202) de l'arrière-fond osseux (200) pour bloquer leurs mouvements relatifs.

## Revendications

1. Calotte d'ancrage (1) dans l'os acétabulaire (2) comprenant plusieurs moyens d'ancrage osseux coopérant, par leur forme spécifique adaptée aux efforts auxquels ils s'opposent, à la fixation initiale puis à long terme, notamment:
- Au moins une plaque (10) disposée radialement à l'équateur (61) de ladite calotte (1) destinée à recevoir un moyen de fixation (11) au bord de l'os acétabulaire (2),
- Au moins un orifice (12) traversant ladite calotte (1), fermé par un obturateur (13) et destiné à recevoir après démontage dudit obturateur (13), un ergot (14) pénétrant dans l'arrière-fond (200) de l'os acétabulaire (2),
- Une partie de contact annulaire (3) dont les aspérités en relief disposées selon plusieurs bandes (31) sensiblement parallèles au plan d'ouverture sont formées par des gorges radiales (32),
**caractérisé par**
- Une partie d'appui polaire en forme de portion de sphère (7) oblique par rapport au dit plan d'ouverture (6), et orientée perpendiculairement à la direction de la résultante (8) des forces musculaires (81 ) et de gravité (82),
- Une série de nervures (9) anti rotationnelles disposées radialement et en relief par rapport à ladite partie d'appui polaire (7),
et en ce que lesdites gorges radiales sont dirigées vers le pole d'appui (7) et sont alignées de façon à converger vers le pôle d'appui (7) de manière à prolonger l'effet directionnel des nervures (9).

2. Calotte d'ancrage (1) dans l'os acétabulaire (2) selon la revendication 1 comprenant une face interne globalement cylindro sphérique concave.

3. Calotte d'ancrage (1) dans l'os acétabulaire (2) selon les revendications 1 et 2 présentant sur la face convexe d'ancrage osseux un traitement de surface de préférence agressif, du type corindonage à titre d'exemple, pour permettre l'attachement de l'os périphérique.

4. Calotte d'ancrage (1) dans l'os acétabulaire (2) selon les revendications 1 à 3 présentant sur la face convexe d'ancrage osseux une apposition de ciment bio actif pour accélérer le processus de cicatrisation des tissus osseux périphériques.

5. Calotte d'ancrage (1) dans l'os acétabulaire (2) selon les revendications 1 à 4 comprenant au moins 2 ergots (14) pénétrant dans les cornes (201 et 202) de l'arrière-fond osseux (200) pour bloquer leurs mouvements relatifs.

6. Calotte d'ancrage (1) dans l'os acétabulaire (2) selon les revendications 1 à 5 destinée à servir de surface de glissement pour une coquille (16) articulée sur une prothèse fémorale (17).

7. Calotte d'ancrage (1) dans l'os acétabulaire (2) selon les revendications 1 et 2 présentant au moins une plaque (10) disposée à la périphérie de la dite calotte (1), de préférence à distance de son plan d'ouverture (6), à la jonction de la zone cylindrique lisse (62) et de la zone de contact annulaire (3), afin d'augmenter la stabilité des pièces articulaires fémorales (16), par le débord osseux de ladite zone lisse (62) en dessous de l'équateur, cette plaque étant de préférence inclinée vers le pôle d'appui (7) de manière à s'adapter au rebord osseux de la cavité acétabulaire (2) sans besoin de modelage in situ.

## Patentansprüche

1. Verankerungskalotte (1) zur Verankerung im Hüftgelenkspfannenknochen (2), mit mehreren Knochenverankerungsmitteln, die durch ihre an die Kräfte, denen sie entgegenwirken, spezifisch angepasste Form von der anfänglichen Befestigung bis zur Langzeitbefestigung zusammenwirken,
insbesondere
- zumindest eine Platte (10), die radial zum Äquator (61) der Kalotte (1) angeordnet ist, die dazu bestimmt ist, ein Befestigungsmittel (11) zur Befestigung am Rand des Hüftgelenkspfannenknochens (2) aufzunehmen,
- zumindest eine Öffnung (12), die durch die Kalotte (1) hindurchgeht, die durch einen Verschluss (13) verschlossen ist und dazu bestimmt ist, nach Abnahme des Verschlusses (13) einen Zapfen (14) aufzunehmen, der in die Grube (200) des Hüftgelenkspfannenknochens (2) eindringt,
- ein ringförmiger Kontaktbereich (3), dessen erhabenen Unebenheiten, die entlang mehrerer Bänder (31) angeordnet sind, die etwa parallel zur Öffnungsebene verlaufen, durch radiale Nuten (32) gebildet sind, **gekennzeichnet durch**
- einen polaren Abstützbereich in Form eines Kugelabschnitts (7), der bezüglich der Öffnungsebene (6) schräg und senkrecht zur Richtung der Resultierenden (8) aus den Muskelkräften (81) und der Schwerkraft (82) orientiert ist,
- eine Reihe von Antiverdreh-Rippen (9), die bezüglich dem polaren Abstützbereich (7) radial und erhaben angeordnet sind, und dass die radialen Nuten zum Abstützpol (7) hin gerichtet und derart ausgerichtet sind, dass sie zum Abstützpol (7) konvergieren, derart, dass sie die Richtungswirkung der Rippen (9) verlängern.

2. Verankerungskalotte (1) zur Verankerung im Hüftgelenkspfannenknochen (2) nach Anspruch 1, mit einer Innenseite, die in ihrer Gesamtheit zylindrisch - sphärisch konkav ist.

3. Verankerungskalotte (1) zur Verankerung im Hüftgelenkspfannenknochen (2) nach den Ansprüchen 1 und 2, die auf ihrer konvexen Knochenverankerungsseite eine vorzugsweise angreifende Oberflächenbehandlung aufweist, beispielsweise von der Art einer Behandlung mit Korund, um die Anlagerung des umgebenden Knochens zu ermöglichen.

4. Verankerungskalotte (1) zur Verankerung im Hüftgelenkspfannenknochen (2) nach den Ansprüchen 1 bis 3, die auf der konvexen Knochenverankerungsseite eine Apposition aus bioaktivem Zement aufweist, um den Narbenbildungsprozess der umliegenden Knochengewebe zu beschleunigen.

5. Verankerungskalotte (1) zur Verankerung im Hüftgelenkspfannenknochen (2) nach den Ansprüchen 1 bis 4, mit zumindest zwei Zapfen (14), die in die Hörner (201 und 202) der Knochengrube (200) eindringen, um ihre Relativbewegung zu blockieren.

6. Verankerungskalotte (1) zur Verankerung im Hüftgelenkspfannenknochen (2) nach den Ansprüchen 1 bis 5, die dazu bestimmt ist, als Gleitfläche für eine Lagerschale (16) zu dienen, die an einer Oberschenkelprothese (17) angelenkt ist.

7. Verankerungskalotte (1) zur Verankerung im Hüftelenkspfannenknochen (2) nach den Ansprüchen 1 und 2, die zumindest ein Platte (10) aufweist, die am Umfang der Kalotte (1), vorzugsweise im Abstand von ihrer Öffnungsebene (6), an der Verbindungsstelle der glatten zylindrischen Zone (62) und der ringförmigen Kontaktzone (3) angeordnet ist, um die Stabilität der gelenkigen Oberschenkelteile (16) durch das Überstehen von Knochen von der glatten Zone (62) unterhalb des Äquators zu erhöhen, wobei diese Platte vorzugsweise zu dem Abstützpol (7) geneigt ist, derart, dass sie sich an den überstehenden Knochenrand des Hüftgelenkspfannenhohlraums (2) ohne die Notwendigkeit einer Modellage in situ anpasst.

## Claims

1. Cup (1) for anchoring in the acetabular bone (2), comprising several bone-anchoring means which, by virtue of their specific form adapted to the stresses which they counter, cooperate in the initial fixation, then the long-term fixation, in particular:
- at least one plate (10) arranged radially at the equator (61) of said cup (1) and intended to receive a fixation means (11) at the margin of the acetabular bone (2),
- at least one orifice (12) passing through said cup (1), closed by an obturator (13) and intended to receive, after removal of said obturator (13), a pin (14) which penetrates into the innermost depth (200) of the acetabular bone (2),
- an annular contact part (3) whose raised rough areas, arranged in several bands (31) substantially parallel to the plane of opening, are formed by radial grooves (32),
**characterized by**
- a polar support part in the form of a portion of a sphere (7) which is oblique relative to said opening plane (6) and is oriented perpendicular to the direction of the resultant (8) of the muscle forces (81) and gravity (82),
- a series of anti-rotation ribs (9) which are arranged radially and are in relief with respect to said polar support part (7),
and in that said radial grooves are directed towards the support pole (7) and are aligned in such a way as to converge towards the support pole (7) so as to prolong the directional effect of the ribs (9).

2. Cup (1) for anchoring in the acetabular bone (2) according to Claim 1, comprising an inner face which overall is concave and cylindro-spherical.

3. Cup (1) for anchoring in the acetabular bone (2) according to Claims 1 and 2, having, on the convex bone-anchoring face, a surface treatment, preferably aggressive, of the corundum type for example, to permit attachment of the peripheral bone.

4. Cup (1) for anchoring in the acetabular bone (2) according to Claims 1 to 3, having, on the convex bone-anchoring face, a coating of bioactive cement for accelerating the process of cicatrization of the peripheral bone tissues.

5. Cup (1) for anchoring in the acetabular bone (2) according to Claims 1 to 4, comprising at least 2 pins (14) penetrating into the horns (201 and 202) of the innermost osseous depth (200) in order to block their relative movements.

6. Cup (1) for anchoring in the acetabular bone (2) according to Claims 1 to 5, intended to serve as a slide surface for a ball (16) articulated on a femoral prosthesis (17).

7. Cup (1) for anchoring in the acetabular bone (2) according to Claims 1 and 2, having at least one plate (10) arranged at the periphery of said cup (1), preferably at a distance from its opening plane (6), at the junction of the smooth cylindrical zone (62) and the annular contact zone (3), so as to increase the stability of the femoral articulation components (16) by bone extending beyond said smooth zone (62) below the equator, this plate preferably being inclined towards the support pole (7) so as to adapt to the osseous margin of the acetabular cavity (2) without the need for in situ modelling.
